(19) Europäisches Patentamt
European Patent Office
Office européen des brevets

(11) Numéro de publication : **0 125 975**
**B1**

# (12) FASCICULE DE BREVET EUROPÉEN

(45) Date de publication du fascicule du brevet : **26.03.86**

(21) Numéro de dépôt : **84400889.6**

(22) Date de dépôt : **02.05.84**

(51) Int. Cl.4 : **C 07 C119/14**, C 07 D307/12, A 61 K 31/22, A 61 K 31/34

## (54) Diphénylazométhines portant une chaîne estérifiée, leur préparation et leur application en thérapeutique.

(30) Priorité : **11.05.83 FR 8307863**

(43) Date de publication de la demande : **21.11.84 Bulletin 84/47**

(45) Mention de la délivrance du brevet : **26.03.86 Bulletin 86/13**

(84) Etats contractants désignés : **AT BE CH DE FR GB IT LI LU NL SE**

(56) Documents cités : **FR-A- 2 319 338**

(73) Titulaire : **SYNTHELABO**
**58, rue de la Glacière**
**F-75621 Paris Cedex 13 (FR)**

(72) Inventeur : **Kaplan, Jean-Pierre**
**20, rue Arnoux**
**F-92340 Bourg-la-Reine (FR)**

(74) Mandataire : **Thouret-Lemaitre, Elisabeth et al**
**Service Brevets - SYNTHELABO 58, rue de la Glacière**
**F-75621 Paris Cedex 13 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 125 975 B1

Jouve, 18, rue St-Denis, 75001 Paris, France

## Description

La présente invention concerne des diphénylazométhines portant une chaîne estérifiée, leur préparation et leur application en thérapeutique.

Les composés de l'invention répondent à la formule

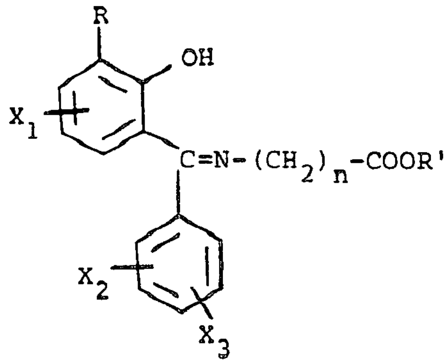

(I)

dans laquelle

$X_1$, $X_2$ et $X_3$ représentent chacun, indépendamment l'un de l'autre, un atome d'hydrogène ou d'halogène, ou un radical $(C_{1-6})$alkyle,

R représente un radical $(C_{1-6})$alkyle droit ou ramifié,

n représente un nombre entier allant de 1 à 10, et

R' représente un radical $(C_{1-6})$cycloalkyl-$(C_{1-4})$alkyle, un radical $(C_{3-7})$cycloalkyle, un radical $(C_{2-6})$ alcényle, un radical $(C_{3-6})$alcynyle, un radical $(C_{1-4})$alcoxy-$(C_{1-4})$alkyle, un radical hydroxy-$(C_{1-4})$ alkyle, un radical $(C_{1-4})$alcoxy-$[(C_{1-4})$alcoxy$]_m$-$(C_{1-4})$alkyle, dans lequel m est un nombre entier allant de 1 à 3, un radical $(C_{1-4})$alcoxycarbonyl-$(C_{1-4})$alkyle, un radical tétrahydrofurylméthyle, un radical phényl-$(C_{1-4})$alkyle pouvant porter un atome d'halogène ou un radical $(C_{1-4})$alcoxy.

Les composés préférés de l'invention sont ceux qui répondent à la formule

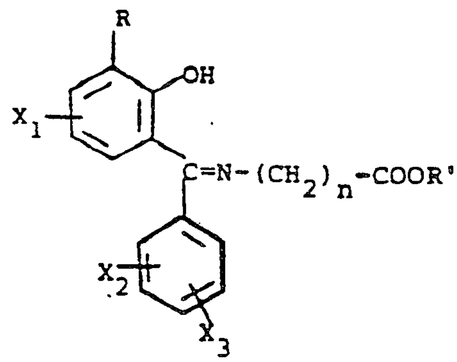

dans laquelle

$X_1$, $X_2$ et $X_3$ représentent chacun indépendamment l'un de l'autre un atome d'hydrogène, un atome de chlore ou le radical méthyle, éthyle ou propyle,

n représente un nombre entier égal à 3, 4 ou 5,

R représente le radical méthyle, éthyle, n-propyle ou isobutyle,

R' représente un radical $(C_{1-6})$alkyle droit ou ramifié, cyclopropylméthyle, cyclopentylméthyle, cyclohexylméthyle, allyle, hydroxyéthyle, $(C_{1-4})$alcoxy-éthyle, éthoxycarbonylméthyle, tétrahydrofurylméthyle, (méthoxy-2 éthoxy)-2 éthyle, [(méthoxy-2 éthoxy)-2 éthoxy]-2 éthyle, cyclohexyle, cyclopentyle, benzyle, propargyle, méthoxy-benzyle ou iso-pentène-2yle ; particulièrement les composés dans lesquels

$X_1$, $X_2$ et $X_3$ représentent chacun indépendamment l'un de l'autre un atome d'hydrogène, un atome de chlore ou le radical méthyle,

n est un nombre entier égal à 3, 4 ou 5,

R représente le radical méthyle, éthyle ou n-propyle, et

R' représente le radical méthyle, éthyle, propyle, allyle ou éthoxy-éthyle,

et plus particulièrement les composés qui répondent à la formule

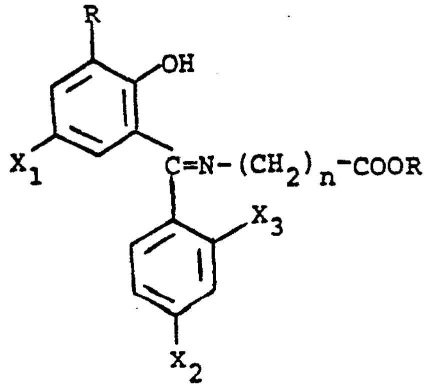

dans laquelle les radicaux ont les significations données ci-dessus ; parmi ces derniers les composés de choix sont ceux pour lesquels $X_1$ est un atome de chlore, $X_2$ est un atome de chlore ou le radical méthyle et $X_3$ est un atome d'hydrogène.

Selon l'invention, on peut préparer les composés selon les trois méthodes suivantes :

méthode A

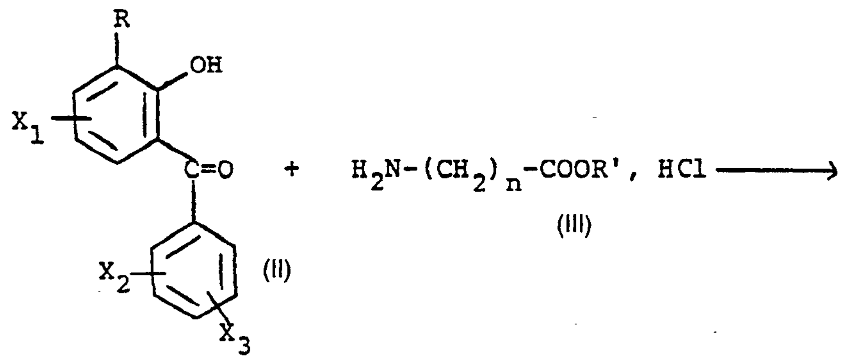

(II) (III) (I)

méthode B

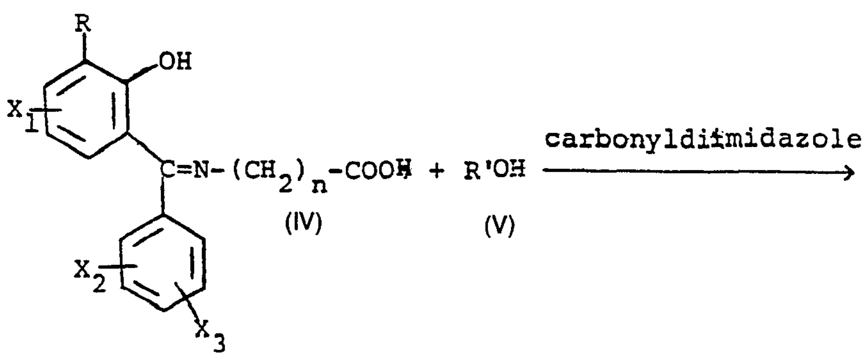


(IV) (V) (I)

méthode C

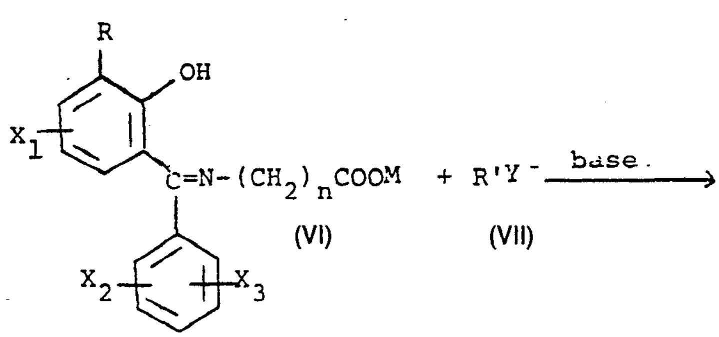


(VI) (VII) (I)

M = métal alcalin

Y = halogène ou groupe labile tel que mésyle ou tosyle.

Selon la méthode A, on fait réagir une cétone (II), décrite dans le brevet 82 21559 ou 82 19981 de la demanderesse avec le chlorhydrate d'un ester de l'acide ω-amino-alcanoïque (III), dans un solvant tel qu'un alcool (méthanol ou éthanol), à une température de 60 à 100 °C.

Selon la méthode B, on fait réagir un acide (IV), décrit dans le brevet 81 21559 ou 82 19981 de la demanderesse, avec un alcool R'OH (V), en présence de carbonyldiimidazole, dans un solvant tel que le tétrahydrofuranne, à une température de 60 à 140 °C.

Selon la méthode C, on fait réagir un sel alcalin de l'acide (VI), décrit dans le brevet 81 21559 ou 82 19981 de la demanderesse, avec un composé R'Y(VII) dans un solvant tel que le diméthylsulfoxyde anhydre, en présence d'une base telle que la triéthylamine, à une température de 50 à 150 °C.

Les chlorhydrates des esters des acides ω-amino-alcanoïques (III) peuvent être préparés à partir de l'acide ω-amino-alcanoïque par réaction avec l'alcool correspondant R'OH en présence de $SOCl_2$.

Les alcools R'OH (V) et les composés R'Y (VII) sont décrits dans la littérature.

Les exemples suivants illustrent l'invention.

Les microanalyses et les spectres IR, UV et RMN confirment la structure des composés.

Exemple 1

{[(chloro-5 hydroxy-2n-propyl-3 phényl)(chloro-4 phényl)méthylène]amino}-4 butanoate d'éthyle.

($X_1$ = 5-Cl, $X_2$ = 4-Cl, $X_3$ = H, n = 3, R = n-$C_3H_7$, R' = $C_2H_5$)

1. On porte à 50-60 °C, dans un bain d'huile, tout en agitant, une suspension de 206 g (2 moles) d'acide amino-4 butanoïque et 1 200 $cm^3$ d'éthanol. On ajoute en une heure, en portant et maintenant 3 h à la température du reflux, 252 ml de $SOCl_2$.

On amène le mélange à siccité et lave le résidu avec 2 fois 500 $cm^3$ d'éther.

Après purification de la solution du résidu dans de l'éthanol à l'aide de charbon végétal et lavage à l'éther, on obtient des cristaux blancs hygroscopiques.

F = 72 °C

2. On évapore à siccité un mélange de 6 g (0,019 4 mole) de (chloro-5 hydroxy-2 n-propyl-3 phényl) (chloro-4 phényl) méthanone, 4,06 g (0,024 2 mole) de chlorhydrate d'amino-4 butanoate d'éthyle et 4,06 g (0,048 3 mole) de bicarbonate de sodium dans 600 ml d'éthanol. On agite le mélange réactionnel sous pression réduite, à 100 °C, pendant 15 mn.

On évapore ensuite 3 fois 500 ml d'éthanol. On reprend le résidu par 300 ml de $CH_2Cl_2$ et 200 ml d'eau, on laisse décanter le mélange réactionnel, on sépare la phase organique et on la sèche sur $MgSO_4$. On évapore à siccité. On purifie sur une colonne de silice en éluant le mélange avec 2,5 l de $CH_2Cl_2$.

On sèche les fractions sur $MgSO_4$, filtre et évapore à siccité. On fait cristalliser le résidu dans 50 ml d'éther. On filtre, essore et sèche les cristaux au dessiccateur en présence de $P_2O_5$.

F = 57-58 °C.

Exemple 2

{[(chloro-5 hydroxy-2 méthyl-3 phényl)(chloro-4 phényl)méthylène]amino}-5 pentanoate d'éthyle.

[$X_1$ = 5-Cl, $X_2$ = 4-Cl, $X_3$ = H, n = 4, R = $CH_3$, R' = $C_2H_5$]

Dans un ballon de 1 l on introduit 15 g (0,053 3 mole) de (chloro-5 hydroxy-2 méthyl-3 phényl)(chloro-4 phényl) méthanone, 2,96 g (0,054 7 mole) de méthylate de sodium et 6,25 g (0,053 3 mole) d'acide amino-5 pentanoïque. On ajoute 400 ml de toluène et 100 ml de méthanol, on chauffe progressivement et distille l'azéotrope formé puis chauffe à la température de reflux du toluène en utilisant un appareil Dean-Stark pendant 6 heures.

On évapore à sec, triture dans de l'éther et filtre le solide. On introduit le solide dans de l'eau et acidifie à pH = 4 avec de l'acide citrique. On filtre le solide, le dissout dans du chloroforme, sèche sur sulfate de magnésium et évapore. On recristallise le composé dans un mélange cyclohexane/benzène. On dissout à nouveau le solide dans du chlorure de méthylène et passe la solution sur une colonne de silice en éluant avec du chlorure de méthylène puis avec un mélange chlorure de méthylène/acétate d'éthyle 80/20. Après évaporation, on obtient un solide que l'on sèche.

F = 110-111 °C

On dissout 5 g (0,013 1 mole) de l'acide {[(chloro-5 hydroxy-2 méthyl-3 phényl)(chloro-4 phényl)méthylène]amino}-5 pentanoïque, obtenu précédemment, dans 100 ml de tétrahydrofuranne. On ajoute ensuite 3,5 g (0,021 5 mole) de carbonyldiimidazole et on agite le mélange réactionnel à la température ambiante pendant une heure. On ajoute à nouveau 3,5 g (0,021 5 mole) de carbonyldiimidazole et poursuit l'agitation pendant 1 h. On évapore à siccité et reprend le résidu par 200 ml d'éther et 200 ml d'eau ; on laisse décanter le mélange réactionnel, on sépare la phase organique, on la sèche sur $MgSO_4$, on filtre $MgSO_4$ et on évapore le filtrat à siccité.

Après addition de 30 ml d'hexane au résidu, le produit cristallise.

Après filtration et recristallisation, on sèche les cristaux.

F = 73-74 °C

## Exemple 3

{[(chloro-5 hydroxy-2 méthyl-3 phényl) (chloro-4 phényl)méthylène]amino}-4 butanoate de cyclohexylméthyle.

[$X_1$=5-Cl, $X_2$=4-Cl, $X_3$=H, n=3, R=$CH_3$, R'= 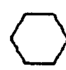 -$CH_2$ ]

A 5,8 g (0,001 5 mole) du sel de sodium de l'acide (chloro-5 hydroxy-2 méthyl-3 phényl) (chloro-4 phényl)méthylène amino-4 butanoïque dans 100 ml de DMSO anhydre, on ajoute 2,5 ml (d = 1,27 soit 0,018 mole), de bromure de cyclohexylméthyle et 2 gouttes de triéthylamine ; on chauffe le mélange réactionnel à 100 °C, en agitant pendant 4 heures. Puis on évapore sous vide le DMSO et partage le résidu entre 100 ml d'éther et 100 ml d'eau. On décante, sèche la phase éthérée sur $MgSO_4$, filtre et évapore à siccité le filtrat. On obtient une huile qui cristallise dans 40 ml de pentane. On filtre les cristaux, lave avec 10 ml de pentane, essore et sèche au dessiccateur chauffant à 40°, en présence de $P_2O_5$.

F = 53-54 °C

(Voir Tableau page 6)

Tableau

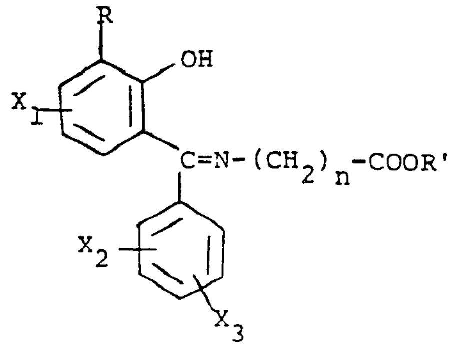

| Composé | $X_1$ | $X_2$ | $X_3$ | R | R' | n | F(°C) ou $n_D$ |
|---|---|---|---|---|---|---|---|
| 1 | 5-Cl | 4-Cl | H | $CH_3$ | $CH_3$ | 3 | $n_D^{20}$=1,5889 |
| 2 | 5-Cl | 3-$CH_3$ | H | $CH_3$ | $CH_3$ | 3 | F=64-65 |
| 3 | 5-Cl | 4-Cl | H | n-$C_3H_7$ | $CH_3$ | 3 | $n_D^{21,5}$=1,5820 |
| 4 | 5-Cl | H | H | $CH_3$ | $CH_3$ | 3 | F=73-74 |
| 5 | 5-$CH_3$ | 4-Cl | H | $CH_3$ | $CH_3$ | 3 | F=63-64 |
| 6 | 5-Cl | 4-Cl | H | $CH_3$ | $C_2H_5$ | 3 | F=52-53 |
| 7 | 5-Cl | 4-$CH_3$ | H | $(CH_3)_2$-CH-$CH_2$- | $CH_3$ | 3 | $n_D^{22,5}$=1,5669 |
| 8 | 5-Cl | 4-$C_2H_5$ | H | $C_2H_5$ | $CH_3$ | 3 | $n_D^{23}$=1,5756 |
| 9 | 5-Cl | 4-Cl | H | $CH_3$ | 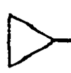-$CH_2$ | 3 | F=-82-83 |
| 10 | 5-Cl | 4-Cl | H | $CH_3$ | $C_2H_5$ | 1 | F=108-109 |
| 11 | 5-Cl | 4-Cl | H | $CH_3$ | n$C_4H_9$ | 3 | F=53-54 |
| 12 | 5-Cl | 4-$CH_3$ | H | $CH_3$ | $CH_3$ | 3 | $n_D^{23}$=1,5884 |
| 13 | 5-Cl | 4-Cl | H | $CH_3$ | $CH_3$ | 2 | F=72-73 |
| 14 | 5-Cl | 4-Cl | H | n-$C_3H_7$ | $C_2H_5$ | 3 | F=57-58 |
| 15 | 5-Cl | 4-Cl | H | $CH_3$ | n$C_6H_{13}$ | 3 | $n_D^{22,5}$=1,6064 |
| 16 | 5-Cl | 3-$CH_3$ | H | $CH_3$ | $C_2H_5$ | 3 | $n_D^{21}$=1,5800 |
| 17 | 5-Cl | 4-Cl | H | $CH_3$ | $C_2H_5$ | 4 | F=73-74 |

Tableau (suite 1)

| Composé | $X_1$ | $X_2$ | $X_3$ | R | R' | n | F(°C) ou $n_D$ |
|---|---|---|---|---|---|---|---|
| 18 | 5-Cl | 4-Cl | H | $CH_3$ | $CH_3$ | 1 | F=133-134 |
| 19 | 5-Cl | 4-Cl | H | $CH_3$ | $nC_3H_7$ | 3 | F=54-56 |
| 20 | 5-Cl | 4-Cl | H | $CH_3$ | $CH_2-CH=CH_2$ | 3 | F=57-58 |
| 21 | 5-Cl | 4-Cl | H | $CH_3$ | $CH_3$ | 4 | F=61-62 |
| 22 | 5-$CH_3$ | 4-Cl | H | $CH_3$ | $C_2H_5$ | 3 | F=52-53 |
| 23 | 5-Cl | 4-Cl | H | $CH_3$ | $CH_3$ | 5 | F=39-40 |
| 24 | 5-Cl | 4-Cl | H | $CH_3$ | $C_2H_5$ | 2 | F=55-56 |
| 25 | 5-Cl | 4-Cl | H | $CH_3$ | 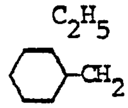 | 3 | F=53-54 |
| 26 | 5-Cl | 4-Cl | H | $nC_3H_7$ | $CH_3$ | 2 | F=69-70 |
| 27 | 5-Cl | 4-Cl | H | $nC_3H_7$ | $C_2H_5$ | 2 | F=71-72 |
| 28 | 5-Cl | 4-Cl | H | $CH_3$ | $nC_5H_{11}$ | 3 | $n_D^{21,5}$=1,5669 |
| 29 | 5-Cl | 4-Cl | H | $CH_3$ | $C_2H_5$ | 5 | F=34-35 |
| 30 | 5-Cl | 4-$CH_3$ | H | $(CH_3)_2-CH-CH_2$ | $C_2H_5$ | 3 | $n_D^{21}$=1,5609 |
| 31 | 5-Cl | 4-$C_2H_5$ | H | $C_2H_5$ | $C_2H_5$ | 3 | $n_D^{21}$=1,5669 |
| 32 | 5-Cl | 4-Cl | H | $CH_3$ | $CH_2CH_2OH$ | 3 | F=74-75 |
| 33 | 5-Cl | 4-Cl | H | $CH_3$ | $CH_2CH_2OCH_2CH_3$ | 3 | F=35-36 |
| 34 | 5-Cl | 4-Cl | H | $CH_3$ | $CH_2CH_2OCH_3$ | 3 | F=34-35 |
| 35 | 5-Cl | 4-Cl | H | $CH_3$ | $CH_2CH_2OC_4H_9$ | 3 | $n_D^{21,5}$=1,5643 |
| 36 | 5-Cl | 4-$CH_3$ | H | $CH_3$ | $C_2H_5$ | 3 | F=57-58 |
| 37 | 5-Cl | 4-Cl | H | $CH_3$ | $CH_2-COOC_2H_5$ | 3 | $n_D^{22}$=1,5700 |
| 38 | 5-Cl | 4-Cl | H | $CH_3$ | 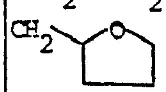 | 3 | F=77-78 |
| 39 | 5-Cl | 4-Cl | H | $CH_3$ | $[(CH_2)_2-O]-_2CH_3$ | 3 | F=30-31 |
| 40 | 5-Cl | 4-Cl | H | $CH_3$ | 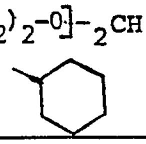 | 3 | F=76-77 |

Tableau (suite 2)

| Composé | $X_1$ | $X_2$ | $X_3$ | R | R' | n | F(°C) ou $n_D$ |
|---|---|---|---|---|---|---|---|
| 41 | 5-Cl | 4-$CH_3$ | 2-$CH_3$ | $CH_3$ | $C_2H_5$ | 3 | $n_D^{20,5}$=1,5743 |
| 42 | 5-Cl | 4-Cl | H | $CH_3$ | $CH_2$- 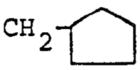 | 3 | $n_D^{21}$= 1,5575 |
| 43 | 5-Cl | 4-Cl | H | $CH_3$ | 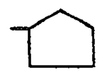 | 3 | F= 95-96 |
| 44 | 5-Cl | 4-$CH_3$ | H | $nC_3H_7$ | $CH_3$ | 3 | $n_D^{21,5}$=1,5774 |
| 45 | 5-Cl | 4-Cl | H | $nC_3H_7$ | $CH_3$ | 4 | F= 64-65 |
| 46 | 5-Cl | 4-Cl | H | $nC_3H_7$ | $C_2H_5$ | 4 | F= 41-42 |
| 47 | 5-Cl | 4-Cl | H | $nC_3H_7$ | $CH_3$ | 5 | F= 40-41 |
| 48 | 5-$CH_3$ | 4-$CH_3$ | 2-$CH_3$ | $CH_3$ | $CH_3$ | 3 | $n_D^{23}$= 1,5710 |
| 49 | 5-Cl | 4-Cl | H | $nC_3H_7$ | $C_2H_5$ | 5 | $n_D^{23}$= 1,5663 |
| 50 | 5-Cl | 4-Cl | H | $CH_3$ | $CH_2$- 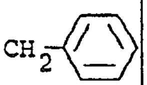 | 3 | F= 101-102 |
| 51 | 5-Cl | 4-$CH_3$ | 2-$CH_3$ | $CH_3$ | $CH_3$ | 3 | $n_D^{23}$= 1,5770 |
| 52 | 5-Cl | 4-Cl | H | $CH_3$ | $CH_2$- 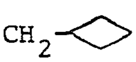 | 3 | F= 102-103 |
| 53 | 5-Cl | 4-Cl | H | $CH_3$ | $-CH_2C{\equiv}CH$ | 3 | F= 70-71 |
| 54 | 5-$CH_3$ | 4-$nC_3H_7$ | H | $C_2H_5$ | $CH_3$ | 3 | $n_D^{21}$= 1,5610 |
| 55 | 5-Cl | 4-Cl | H | $CH_3$ | i-$C_3H_7$ | 3 | $n_D^{21}$= 1,5751 |
| 56 | 5-$CH_3$ | 4-Cl | 2-$CH_3$ | $CH_3$ | $CH_3$ | 3 | $n_D^{22,5}$=1,5810 |
| 57 | 5-Cl | 4-Cl | H | $CH_3$ | 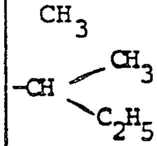 | 3 | $n_D^{21}$= 1,5725 |

Tableau (suite 3)

| Composé | $X_1$ | $X_2$ | $X_3$ | R | R' | n | F(°C) ou $n_D$ |
|---|---|---|---|---|---|---|---|
| 58 | 5-Cl | 4-Cl | H | $CH_3$ | $(CH_2CH_2O)_3CH_3$ | 3 | $n_D^{23}$=1,5559 |
| 59 | 5-Cl | 4-Cl | H | $CH_3$ | 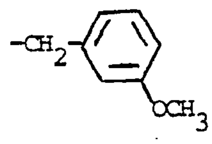 | 3 | F=58-59 |
| 60 | 5-Cl | 4-Cl | H | $CH_3$ | $CH_3$ | 7 | F=39-40 |
| 61 | 5-Cl | 4-Cl | H | $CH_3$ | $CH_3$ | 6 | F=38-39 |
| 62 | 5-Cl | 4-Cl | H | $CH_3$ | $CH_2-CH=C(CH_3)_2$ | 3 | $n_D^{21}$=1,5751 |
| 63 | 5-Cl | 4-Cl | H | $CH_3$ | $iC_4H_9$ | 3 | F=84-85 |
| 64 | 5-Cl | 4-Cl | H | $CH_3$ | $CH_3$ | 8 | F=37-38 |
| 65 | 5-Cl | 4-Cl | H | $CH_3$ | $CH_3$ | 9 | F=54-55 |
| 66 | 5-Cl | 4-Cl | H | $nC_3H_7$ | $CH_2CH_2O-C_2H_5$ | 3 | $n_D^{23}$=1,5663 |
| 67 | 5-Cl | 4-Cl | H | $CH_3$ | $CH_3$ | 10 | F=39-40 |
| 68 | 5-Cl | 4-Cl | H | $nC_3H_7$ | $(CH_2)_2OCH_3$ | 4 | $n_D^{24,5}$=1,5654 |
| 69 | 5-Cl | 4-Cl | H | $nC_3H_7$ | $(CH_2)_2O-C_2H_5$ | 4 | $n_D^{24,5}$=1,5620 |
| 70 | 5-Cl | 4-Cl | H | $nC_3H_7$ | $CH_2-CH=CH_2$ | 4 | 35-36 |
| 71 | 5-Cl | 4-Cl | H | $nC_3H_7$ | $(CH_2)_2OC_2H_5$ | 5 | $n_D^{26,5}$=1,5554 |
| 72 | 5-Cl | 4-$CH_3$ | H | $nC_3H_7$ | $CH_3$ | 4 | 73-74 |
| 73 | 5-Cl | 4-Cl | H | $nC_3H_7$ | $(CH_2)_2OCH_3$ | 5 | $n_D^{26,5}$=1,5634 |
| 74 | 5-Cl | 4-$CH_3$ | H | $nC_3H_7$ | $C_2H_5$ | 4 | 62-63 |

Tableau (suite 4)

| Composé | $X_1$ | $X_2$ | $X_3$ | R | R' | n | F.(°C) ou $n_D$ |
|---|---|---|---|---|---|---|---|
| 75 | 5-Cl | 4-Cl | H | $nC_3H_7$ | $iC_3H_7$ | 4 | 55-56 |
| 76 | 5-Cl | $4-CH_3$ | H | $nC_3H_7$ | $C_2H_5$ | 3 | $n_D^{34}$=1,5635 |
| 77 | 5-Cl | 4-Cl | H | $CH_3$ | $(CH_2)_2OC_2H_5$ | 5 | $n_D^{30}$=1,5558 |
| 78 | 5-Cl | 4-Cl | H | $CH_3$ | $CH(CH_3)CH_2OCH_3$ | 3 | 55-56 |
| 79 | 5-Cl | $4-CH_3$ | H | $nC_3H_7$ | $CH_3$ | 5 | 31-32 |
| 80 | 5-Cl | 4-Cl | H | $CH_3$ | $(CH_2)_2OC_2H_5$ | 4 | $n_D^{26}$=1,5600 |
| 81 | 5-Cl | 4-Cl | H | $CH_3$ | $(CH_2)_3OC_2H_5$ | 3 | $n_D^{28}$=1,5650 |
| 82 | 5-Cl | 4-Cl | H | $CH_3$ | $(CH_2)_2CH(OCH_3)-CH_3$ | 3 | $n_D^{28}$=1,5653 |
| 83 | 5-Cl | $4-CH_3$ | H | $nC_3H_7$ | $(CH_2)_2OC_2H_5$ | 4 | $n_D^{23}$=1,5042 |
| 84 | 5-Cl | 4-Cl | H | $CH_3$ | $CH_2COCH_3$ | 3 | 78,2 |
| 85 | 5-Cl | $4-CH_3$ | H | $CH_3$ | $CH_3$ | 4 | 70-71 |
| 86 | 5-Cl | $4-CH_3$ | H | $CH_3$ | $CH_3$ | 5 | 40-41 |
| 87 | 5-Cl | $4-C_2H_5$ | H | $C_2H_5$ | $CH_3$ | 5 | $n_D^{23}$=1,5693 |
| 88 | 5-Cl | $4-C_2H_5$ | H | $C_2H_5$ | $CH_3$ | 4 | $n_D^{23}$=1,5722 |
| 89 | 5-Cl | 4-Cl | H | $C_2H_5$ | $CH_3$ | 4 | 70-71 |
| 90 | 5-Cl | 4-Cl | H | $C_2H_5$ | $CH_3$ | 5 | $n_D^{21}$=1,5799 |

Les composés de l'invention ont été soumis à des essais pharmacologiques montrant leur activité sur le système nerveux central.

La toxicité aiguë a été déterminée chez la souris par voie orale. La DL 50 (dose léthale 50 %) induisant la mort chez 50 % des animaux est supérieure à 1 000 mg/kg.

L'activité anticonvulsivante des composés a été montrée par l'antagonisme vis-à-vis de la mortalité induite par la bicuculline chez la souris.

La bicuculline est un bloqueur relativement sélectif des récepteurs GABA-ergiques post synaptiques

et ses effets convulsivants et létaux sont antagonisés par les composés élevant le taux GABA cérébral ou possédant une activité GABA-mimétique.

On a évalué la dose active 50 % (DA 50), dose protégeant 50 % des animaux contre l'effet de la bicuculline, des substances étudiées.

La DA 50 des composés de l'invention va de 10 à 200 mg/kg par voie orale.

Les composés de l'invention sont actifs comme anticonvulsivants et possèdent également des propriétés antidépressives, anxiolytiques, analgésiques, antiinflammatoires, antidépressives, antiulcéreuses et antidiskynétiques. Ils sont utilisables en thérapeutique humaine et vétérinaire, entre autres pour le traitement de diverses maladies du système nerveux central, par exemple pour le traitement des dépressions, des psychoses, de certaines maladies neurologiques comme l'épilepsie, la spasticité, la diskynésie.

L'invention comprend, par conséquent, toutes compositions pharmaceutiques renfermant les composés (I) comme principes actifs, en association avec tous excipients appropriés à leur administration, en particulier par voie orale (comprimés, dragées, gélules, capsules, cachets, solutions ou suspensions buvables) ou parentérale.

La posologie quotidienne peut aller de 100 à 4 000 mg.

**Revendications** (pour les Etats contractants : BE, CH, DE, FR, GB, IT, LI, LU, NL, SE)

1. Diphénylazométhines portant une chaîne estérifiée, répondant à la formule (I)

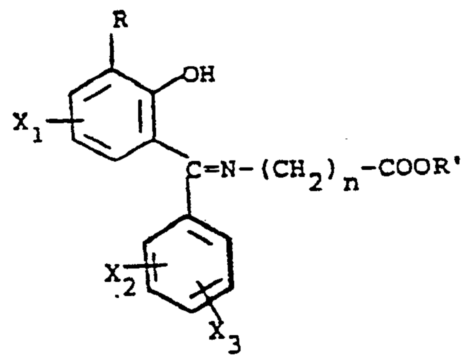

(I)

dans laquelle

$X_1$, $X_2$ et $X_3$ représentent chacun, indépendamment l'un de l'autre, un atome d'hydrogène ou d'halogène, ou un radical $(C_{1-6})$alkyle,

R représente un radical $(C_{1-6})$alkyle droit ou ramifié,

n représente un nombre entier allant de 1 à 10, et

R' représente un radical $(C_{1-6})$alkyle droit ou ramifié, un radical $(C_{3-6})$cycloalkyl-$(C_{1-4})$alkyle, un radical $(C_{3-7})$cycloalkyle, un radical $(C_{2-6})$alcényle, un radical $(C_{3-6})$alcynyle, un radical $(C_{1-4})$alcoxy-$(C_{1-4})$ alkyle, un radical hydroxy-$(C_{1-4})$alkyle, un radical $(C_{1-4})$alcoxy-$[(C_{1-4})$alcoxy$]_m$-$(C_{1-4})$alkyle, dans lequel m est un nombre entier allant de 1 à 3, un radical $(C_{1-4})$alcoxycarbonyl-$(C_{1-4})$alkyle, un radical tétrahydrofurylméthyle, un radical phényl-$(C_{1-4})$alkyle pouvant porter un atome d'halogène ou un radical $(C_{1-4})$alcoxy.

2. Composés selon la revendication 1, caractérisés par le fait que

$X_1$, $X_2$ et $X_3$ représentent chacun indépendamment l'un de l'autre un atome d'hydrogène, un atome de chlore ou le radical méthyle, éthyle ou propyle,

n représente un nombre entier égal à 3, 4 ou 5,

R représente le radical méthyle, éthyle, n-propyle ou isobutyle,

R' représente un radical $(C_{1-6})$alkyle droit ou ramifié, cyclopropylméthyle, cyclopentylméthyle, cyclohexylméthyle, allyle, hydroxyéthyle, $(C_{1-4})$alcoxy-éthyle, éthoxycarbonylméthyle, tétrahydrofurylméthyle, (méthoxy-2 éthoxy)-2 éthyle, [(méthoxy-2 éthoxy)-2 éthoxy]-2 éthyle, cyclohexyle, cyclopentyle, benzyle, propargyle, méthoxy-benzyle ou isopentène-2yle.

3. Composés selon la revendication 2, dans lesquels

$X_1$, $X_2$ et $X_3$ représentent chacun indépendamment l'un de l'autre un atome d'hydrogène, un atome de chlore ou le radical méthyle,

n est un nombre entier égal à 3, 4 ou 5,

R représente le radical méthyle, éthyle ou n-propyle, et

R' représente le radical méthyle, éthyle, propyle, allyle ou éthoxy-éthyle.

4. Composés selon la revendication 3 répondant à la formule

(Voir formule page 12)

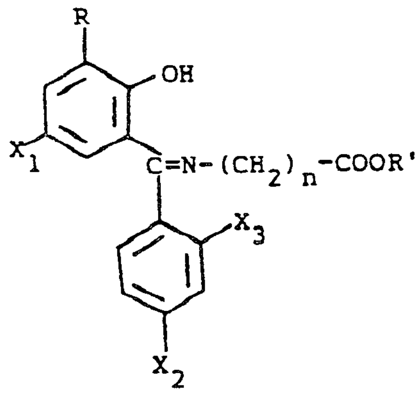

dans laquelle les radicaux ont les significations données dans la revendication 3.

5. Composés selon la revendication 4, caractérisés par le fait que $X_1$ est un atome de chlore, $X_2$ est un atome de chlore ou le radical méthyle et $X_3$ est un atome d'hydrogène, n, R et R' ayant les significations données dans la revendication 3.

6. Procédé de préparation des composés spécifiés dans l'une quelconque des revendications 1 à 5, procédé caractérisé en ce que

soit on fait réagir une benzophénone (II)

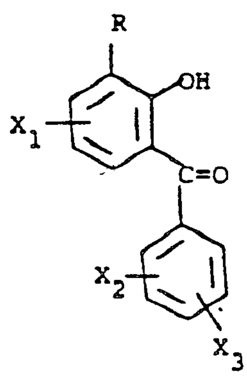

(II)

avec le chlorhydrate d'un ester de l'acide ω-amino-alcanoïque (III)

$$H_2N—(CH_2)_n—COOR', HCl$$

soit on fait réagir un acide (IV)

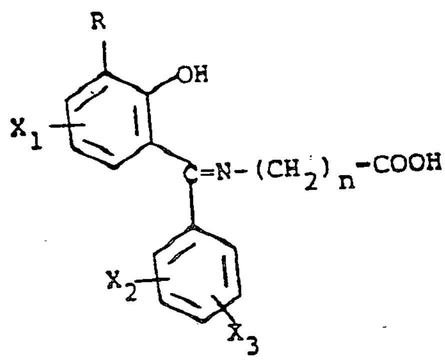

(IV)

avec un alcool R'OH (V) en présence de carbonyldiimidazole,

soit on fait réagir un sel alcalin de l'acide (VI)

(Voir formule page 13)

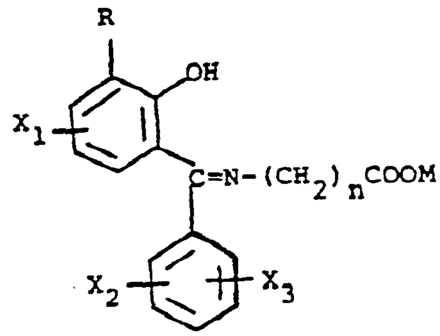

(VI)

avec un composé R'Y (VII) dans lequel Y représente un atome d'halogène ou un groupe labile tel que mésyle ou tosyle, les radicaux $X_1$, $X_2$, $X_3$, n, R et R' ayant les significations données dans les revendications 1 à 5.

7. Médicament caractérisé en ce qu'il contient un composé tel que spécifié dans l'une quelconque des revendications 1 à 5.

8. Composition pharmaceutique caractérisée en ce qu'elle contient un composé tel que spécifié dans l'une quelconque des revendications 1 à 5, en association avec tout excipient approprié.

**Revendication** (pour l'état contractant AT)

Procédé de préparation des diphénylazométhines portant une chaîne estérifiée, répondant à la formule (I)

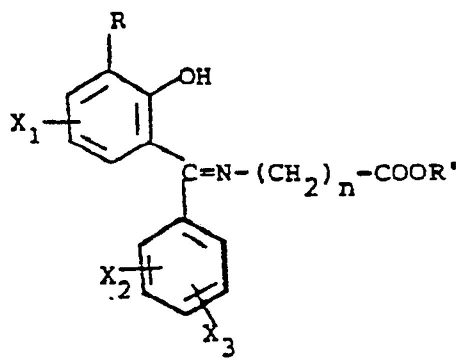

(I)

dans laquelle

$X_1$, $X_2$ et $X_3$ représentent chacun, indépendamment l'un de l'autre, un atome d'hydrogène ou d'halogène, ou un radical $(C_{1-6})$alkyle,

R représente un radical $(C_{1-6})$alkyle droit ou ramifié,

n représente un nombre entier allant de 1 à 10, et

R' représente un radical $(C_{1-6})$alkyle droit ou ramifié, un radical $(C_{3-6})$cycloalkyl-$(C_{1-4})$alkyle, un radical $(C_{3-7})$cycloalkyle, un radical $(C_{2-6})$alcényle, un radical $(C_{3-6})$alcynyle, un radical $(C_{1-4})$alcoxy-$(C_{1-4})$alkyle, un radical hydroxy-$(C_{1-4})$alkyle, un radical $(C_{1-4})$alcoxy-$[(C_{1-4})$alcoxy$]_m$-$(C_{1-4})$alkyle, dans lequel m est un nombre entier allant de 1 à 3, un radical $(C_{1-4})$alcoxycarbonyl-$(C_{1-4})$alkyle, un radical tétrahydrofurylméthyle, un radical phényl-$(C_{1-4})$alkyle pouvant porter un atome d'halogène ou un radical $(C_{1-4})$alcoxy

procédé caractérisé en ce que

soit on fait réagir une benzophénone (II)

(Voir formule page 14)

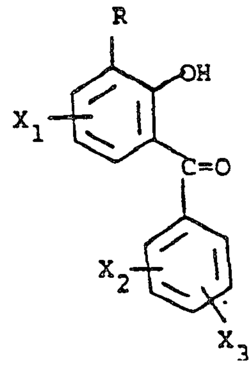

(II)

avec le chlorhydrate d'un ester de l'acide ω-amino-alcanoïque (III)

$$H_2N—(CH_2)_n—COOR', HCl \quad (III)$$

soit on fait réagir un acide (IV)

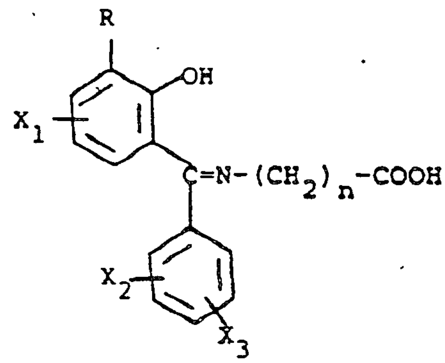

(IV)

avec un alcool R'OH (V) en présence de carbonyldiimidazole,
soit on fait réagir un sel alcalin de l'acide (VI)

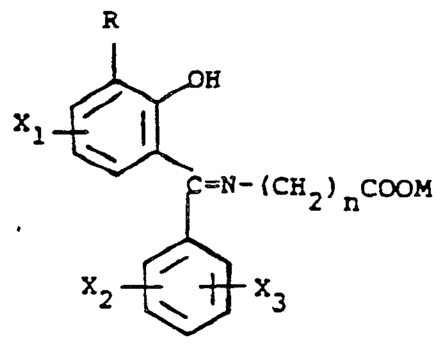

(VI)

avec un composé R'Y (VII) dans lequel Y représente un atome d'halogène ou un groupe labile tel que mésyle ou tosyle, les radicaux $X_1$, $X_2$, $X_3$, n, R et R' ayant les significations données ci-dessus.

**Claims** (for the contracting states : BE, CH, DE, FR, GB, IT, LI, LU, NL, SE)

1. Diphenylazomethines containing an esterified chain and corresponding to the formula (I)

(See formula (I) page 15)

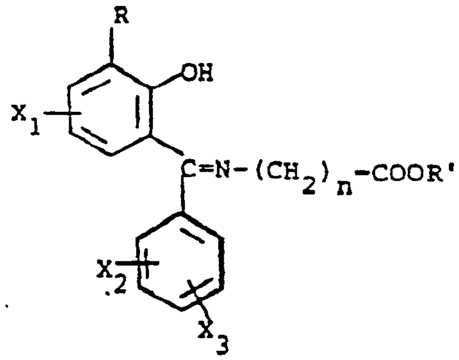

(I)

in which

$X_1$, $X_2$ and $X_3$ independently of one another each represent a hydrogen or halogen atom or a $(C_{1\text{-}6})$ alkyl radical,

R represents a straight-chain or branched $(C_{1\text{-}6})$alkyl radical,

n represents an integer from 1 to 10 and

R' represents a straight-chain or branched $(C_{1\text{-}6})$alkyl radical, a $(C_{3\text{-}6})$cycloalkyl$(C_{1\text{-}4})$alkyl radical, a $(C_{3\text{-}7})$cycloalkyl radical, a $(C_{2\text{-}6})$alkenyl radical, a $(C_{3\text{-}6})$alkynyl radical, a $(C_{1\text{-}4})$alkoxy$(C_{1\text{-}4})$alkyl radical, a hydroxy-$(C_{1\text{-}4})$alkyl radical, a $(C_{1\text{-}4})$alkoxy-$[(C_{1\text{-}4})$alkoxy$]_m$-$(C_{1\text{-}4})$alkyl radical, in which m is an integer from 1 to 3, a $(C_{1\text{-}4})$alkoxycarbonyl$(C_{1\text{-}4})$alkyl radical, a tetrahydrofurylmethyl radical, a phenyl-$(C_{1\text{-}4})$ alkyl radical which can carry a halogen atom, or a $(C_{1\text{-}4})$alkoxy radical.

2. Compounds according to Claim 1, characterised in that

$X_1$, $X_2$ and $X_3$ independently of one another each represent a hydrogen atom, a chlorine atom or the methyl, ethyl or propyl radical,

n represents an integer equal to 3, 4 or 5,

R represents the methyl, ethyl, n-propyl or isobutyl radical and

R' represents a straight-chain or branched $(C_{1\text{-}6})$alkyl radical or a cyclopropylmethyl, cyclopentylmethyl, cyclohexylmethyl, allyl, hydroxyethyl, $(C_{1\text{-}4})$alkoxy-ethyl, ethoxycarbonylmethyl, tetrahydrofuryl-methyl, (methoxy-2 ethoxy)-2 ethyl, [(methoxy-2-ethoxy)-2 ethoxy]-2 ethyl, cyclohexyl, cyclopentyl, benzyl, propargyl, methoxy-benzyl, or iso-penten-2-yl radical.

3. Compounds according to Claim 2, in which

$X_1$, $X_2$ and $X_3$ independently of one another each represent a hydrogen atom, a chlorine atom or the methyl radical,

n is an integer equal to 3, 4 or 5,

R represents the methyl, ethyl or n-propyl radical and

R' represents the methyl, ethyl, propyl, allyl or ethoxyethyl radical.

4. Compounds according to Claim 3, corresponding to the formula

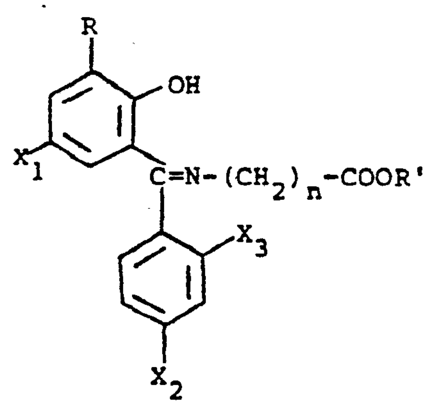

in which the radicals have the meanings given in Claim 3.

5. Compounds according to Claim 4, characterised in that $X_1$ is a chlorine atom, $X_2$ is a chlorine atom or the methyl radical and $X_3$ is a hydrogen atom, n, R and R' having the meanings given in Claim 3.

6. Process for the preparation of the compounds specified in any one of Claims 1 to 5, characterised in that

either a benzophenone (II)

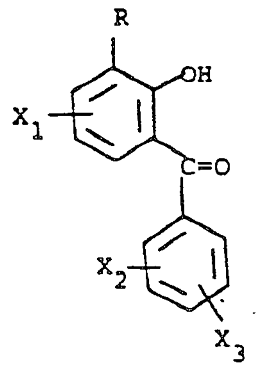

(II)

is reacted with the hydrochloride of an ester of an ω-amino-alkanoic acid (III)

$$H_2N-(CH_2)_n-COOR', HCl$$

or an acide (IV)

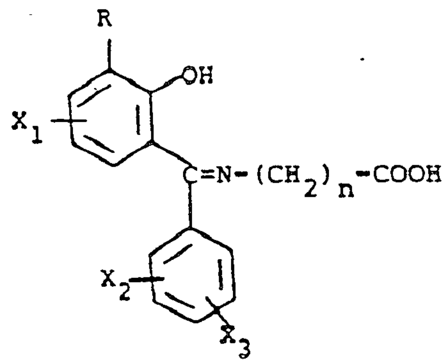

(IV)

is reacted with an alcohol R'OH (V) in the presence of carbonyldiimidazole, or an alkali metal salt of an acid (VI)

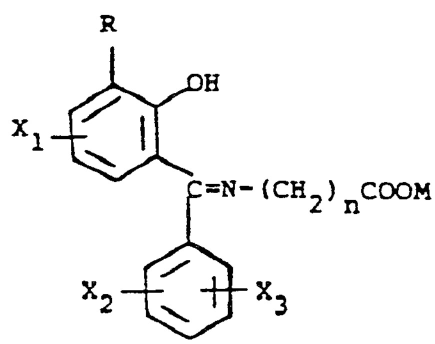

(VI)

is reacted with a compound R'Y (VII), in which Y represents a halogen atom or a labile group, such as mesyl or tosyl, the radicals $X_1$, $X_2$, $X_3$, n, R and R' having the meanings given in Claims 1 to 5.

7. Medicament, characterised in that it contains a compound such as is specified in any one of Claims 1 to 5.

8. Pharmaceutical composition, characterised in that it contains a compound such as is specified in any one of Claims 1 to 5, together with any suitable excipient.

**Claim** (for the contracting state AT)

Process for the preparation of diphenylazomethines containing an esterified chain and corresponding to the formula (1)

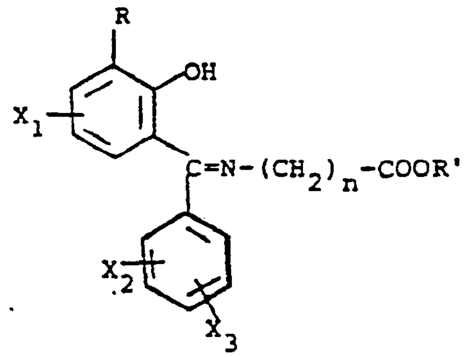

(I)

in which

$X_1$, $X_2$ and $X_3$ independently of one another each represent a hydrogen or halogen atom or a $(C_{1-6})$ alkyl radical,

R represents a straight-chain or branched $(C_{1-6})$alkyl radical,

n represents an integer from 1 to 10 and

R′ represents a straight-chain or branched $(C_{1-6})$alkyl radical, a $(C_{3-6})$cycloalkyl $(C_{1-4})$alkyl radical, a $(C_{3-7})$cycloalkyl radical, a $(C_{2-6})$alkenyl radical, a $(C_{3-6})$alkynyl radical, a $(C_{1-4})$alkoxy-$(C_{1-4})$alkyl radical, a hydroxy-$(C_{1-4})$alkyl radical, a $(C_{1-4})$alkoxy$[(C_{1-4})$alkoxy$]_m$-$(C_{1-4})$alkyl radical, in which m is an integer from 1 to 3, a $(C_{1-4})$alkoxy-carbonyl-$(C_{1-4})$alkyl radical, a tetrahydrofurylmethyl radical, a phenyl-$(C_{1-4})$ alkyl radical which can carry a halogen atom, or a $(C_{1-4})$alkoxy radical,

process characterised in that

either a benzophenone (II)

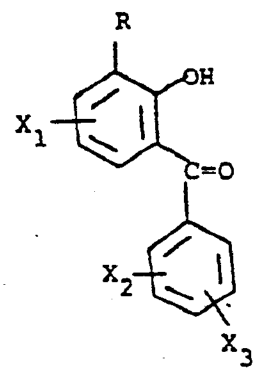

(II)

is reacted with the hydrochloride of an ester of an ω-amino-alkanoic acid (III)

$$H_2N—(CH_2)_n—COOR', HCl \quad (III)$$

or an acid (IV)

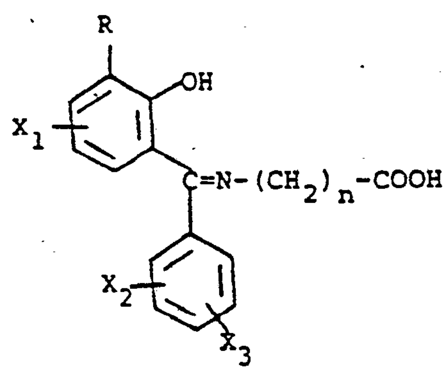

(IV)

is reacted with an alcohol R′OH (V) in the presence of carbonyldiimidazole,

or an alkali metal salt of an acid (VI)

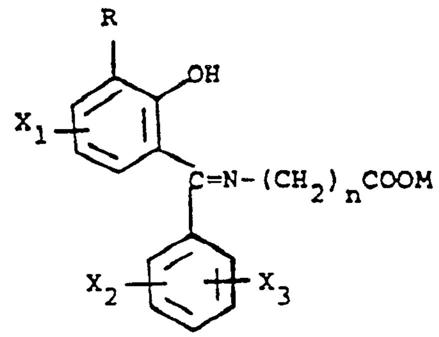

(VI)

is reacted with a compound R'Y (VII), in which Y represents a halogen atom or a labil group, such as mesyl or tosyl, the radicals $X_1$, $X_2$, $X_3$, n, R and R' having the meanings given above.

**Patentansprüche** (für die Vertragsstaaten : BE, CH, DE, FR, GB, IT, LI, LU, NL, SE)

1. Diphenylazomethine, die eine veresterte Seitenkette aufweisen, gemäß der Formel (I)

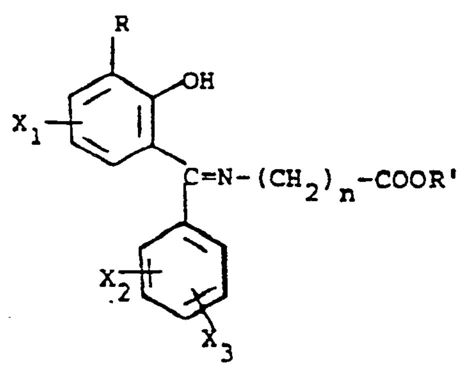

(I)

wobei

$X_1$, $X_2$ und $X_3$ jeweils unabhängig voneinander ein Wasserstoff- oder Halogenatom oder eine $(C_{1-6})$-Alkylgruppe bedeuten,

R eine gerade oder verzweigte $(C_{1-6})$-Alkylgruppe bedeutet,

n eine ganze Zahl von 1 bis 10 bedeutet,

und R' eine gerade oder verzweigte $(C_{1-6})$-Alkylgruppe, eine $(C_{3-6})$-Cycloalkyl-$(C_{1-4})$-alkylgruppe, eine $(C_{3-7})$-Cycloalkylgruppe, eine $(C_{2-6})$-Alkenylgruppe, eine $(C_{3-6})$-Alkinylgruppe, eine $(C_{1-4})$-Alkoxy-$(C_{1-4})$-alkylgruppe, eine Hydroxy-$(C_{1-4})$-alkylgruppe, eine $(C_{1-4})$-Alkoxy($(C_{1-4})$-alkoxy$)_m$-$(C_{1-4})$-alkylgruppe, bei welcher m eine ganze Zahl von 1 bis 3 ist, eine $(C_{1-4})$-Alkoxycarbonyl-$(C_{1-4})$-alkylgruppe, eine Tetrahydrofurylmethylgruppe, eine Phenyl-$(C_{1-4})$-alkylgruppe, die ein Halogenatom oder eine $(C_{1-4})$-Alkoxygruppe tragen kann, bedeutet.

2. Verbindungen nach Anspruch 1, dadurch gekennzeichnet, daß

$X_1$, $X_2$ und $X_3$ jeweils unabhängig voneinander ein Wasserstoffatom, ein Chloratom oder eine Methyl-, Äthyl- oder Propylgruppe bedeuten,

n eine ganze Zahl gleich 3, 4 oder 5 bedeutet,

R eine Methyl-, Äthyl-, n-Propyl- oder Isobutylgruppe bedeutet,

R' eine gerade oder verzweigte $(C_{1-6})$-Alkylgruppe, eine Cyclopropylmethyl-, Cyclopentylmethyl-, Cyclohexylmethyl-, Allyl-, Hydroxyäthyl-, $(C_{1-4})$-Alkoxyäthyl-, Äthoxycarbonylmethyl-, Tetrahydrofurylmethyl-, 2-(2-Methoxyäthoxy)-äthyl-, 2-(2-(2-Methoxyäthoxy)-äthoxy)-äthyl-, cyclohexyl-, Cyclopentyl-, Benzyl-, Propargyl-, Methoxybenzyl- oder Isopenten-2-yl-Gruppe bedeutet.

3. Verbindungen nach Anspruch 2, wobei

$X_1$, $X_2$ und $X_3$ jeweils unabhängig voneinander ein Wasserstoffatom, ein Chloratom oder eine Methylgruppe bedeuten,

n eine ganze Zahl gleich 3, 4 oder 5 bedeutet,

R eine Methyl-, Äthyl- oder n-Propylgruppe bedeutet,

R' eine Methyl-, Äthyl-, Propyl-, Allyl- oder Äthoxyäthyl-Gruppe bedeutet.

4. Verbindungen nach Anspruch 3 gemäß der Formel

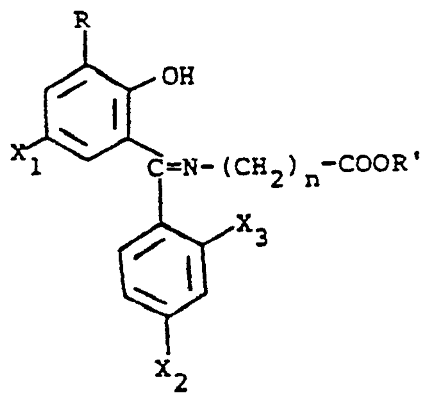

wobei die Gruppen die in Anspruch 3 angegebenen Bedeutungen haben.

5. Verbindungen nach Anspruch 4, dadurch gekennzeichnet, daß $X_1$ ein Chloratom, $X_2$ ein Chloratom oder eine Methylgruppe und $X_3$ ein Wasserstoffatom bedeutet, und n, R und R' die in Anspruch 3 angegebenen Bedeutungen haben.

6. Verfahren zur Herstellung der Verbindungen nach einem der Ansprüche 1 bis 5, dadurch gekennzeichnet, daß

entweder ein Benzophenon (II)

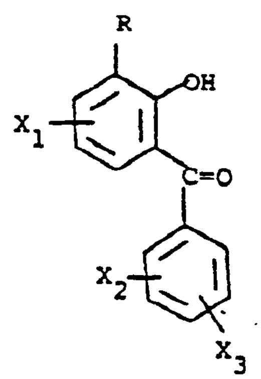

(II)

mit dem Chlorhydrat eines Esters einer ω-Amino-alkansäure (III)

$$H_2N-(CH_2)-COOR', HCl \quad (III)$$

zur Reaktion gebracht wird,

oder eine Säure (IV)

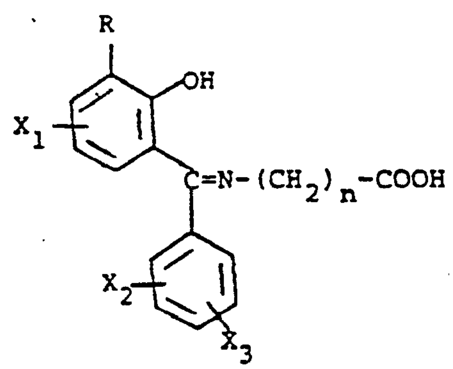

(IV)

mit einem Alkohol R' (OH) (V) in Anwesenheit von Carbonyldiimidazol zur Reaktion gebracht wird,

oder ein Alkalisalz der Säure (VI)

(Siehe Formel Seite 20 f.)

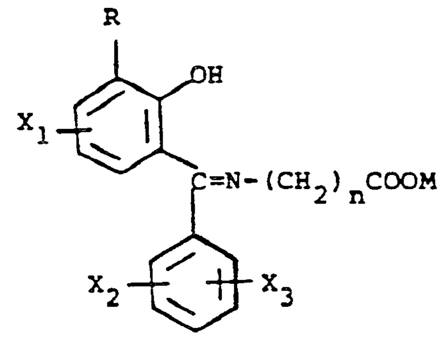

(VI)

mit einer Verbindung R'Y (VII) zur Reaktion gebracht wird, wobei Y ein Halogenatom oder eine labile Gruppe wie Mesyl oder Tosyl bedeutet, und die Gruppen $X_1$, $X_2$, $X_3$, n, R und R' die in den Ansprüchen 1 bis 5 angegebenen Bedeutungen haben.

7. Arzneimittel, dadurch gekennzeichnet, daß es eine Verbindung nach einem der Ansprüche 1 bis 5 enthält.

8. Pharmazeutische Zubereitung, dadurch gekennzeichnet, daß sie eine Verbindung nach einem der Ansprüche 1 bis 5, gemeinsam mit irgendeinem geeigneten Exzipiens enthält.

**Patentanspruch** (für den Vertragsstaat AT)

Verfahren zur Herstellung von Diphenylazomethinen, die eine veresterte Seitenkette aufweisen, gemäß der Formel (I)

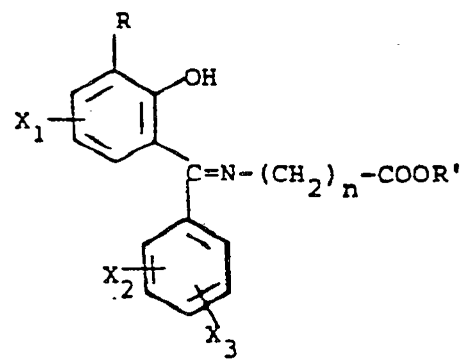

(I)

wobei

$X_1$, $X_2$ und $X_3$ jeweils unabhängig voneinander ein Wasserstoff- oder Halogenatom oder eine $(C_{1-6})$-Alkylgruppe bedeuten,

R eine gerade oder verzweigte $(C_{1-6})$-Alkylgruppe bedeutet,

n eine ganze Zahl von 1 bis 10 bedeutet,

und R' eine gerade oder verzweigte $(C_{1-6})$-Alkylgruppe, eine $(C_{3-6})$-Cycloalkyl-$(C_{1-4})$-alkylgruppe, eine $(C_{3-7})$-Cycloalkylgruppe, eine $(C_{2-6})$-Alkenylgruppe, eine $(C_{3-6})$-Alkinylgruppe, eine $(C_{1-4})$-Alkoxy-$(C_{1-4})$-alkylgruppe, eine Hydroxy-$(C_{1-4})$-alkylgruppe, eine $(C_{1-4})$-Alkoxy-$((C_{1-4})$-alkoxy$)_m$-$(C_{1-4})$-alkylgruppe, bei welcher m eine ganze Zahl von 1 bis 3 ist, eine $(C_{1-4})$-Alkoxycarbonyl-$(C_{1-4})$-alkylgruppe, eine Tetrahydrofurylmethylgruppe, eine Phenyl-$(C_{1-4})$-alkylgruppe, die ein Halogenatom oder eine $(C_{1-4})$-Alkoxygruppe tragen kann, bedeutet, dadurch gekennzeichnet, daß

entweder ein Benzophenon (II)

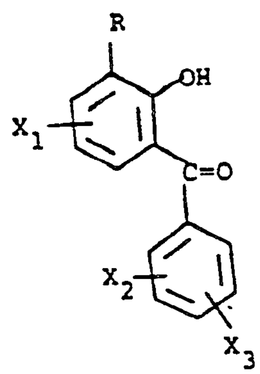

(II)

mit dem Chlorhydrat eines Esters einer ω-Amino-alkansäure (III)

$$H_2N—(CH_2)_n—COOR', HCl \qquad (III)$$

zur Reaktion gebracht wird, oder eine Säure (IV)

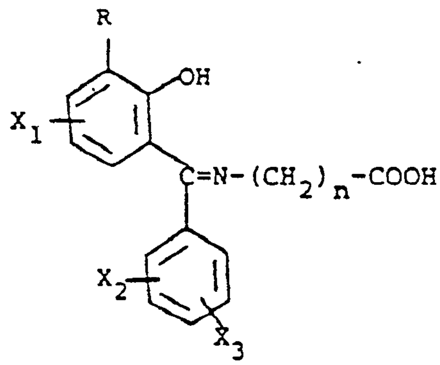

(IV)

mit einem Alkohol R' (OH) (V) in Anwesenheit von Carbonyldiimidazol zur Reaktion gebracht wird, oder ein Alkalisalz der Säure (VI)

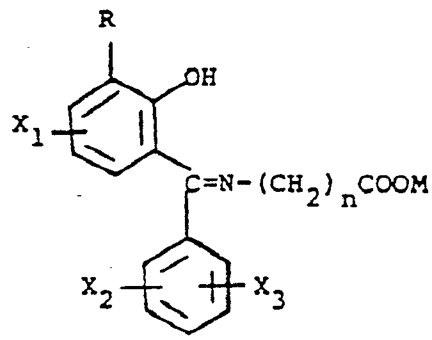

(VI)

mit einer Verbindung R' Y (VII) zur Reaktion gebracht wird, wobei Y ein Halogenatom oder eine labile Gruppe wie Mesyl oder Tosyl bedeutet, und die Gruppen $X_1$, $X_2$, $X_3$, n, R und R' die oben angegebene Bedeutung haben.